# EUROPÄISCHE PATENTSCHRIFT

(11) **EP 1 233 012 B1**
(45) Veröffentlichungstag und Bekanntmachung des Hinweises auf die Patenterteilung: **21.05.2003**
(21) Anmeldenummer: 01103224.0
(22) Anmeldetag: 12.02.2001
(51) Int. Cl.: C07C 51/573, C07C 63/16, B01D 3/14

(54) **Verfahren zur herstellung von spezifikationsgerechtem phthalsäureanhydrid**
Method for producing phthalic anhydride according to specifications
Procédé pour produire de l'anhydride phtalique conforme aux specifications

(43) Veröffentlichungstag der Anmeldung: 21.08.2002
(73) Patentinhaber: BASF AKTIENGESELLSCHAFT, 67056 Ludwigshafen (DE)
(72) Erfinder: Disteldorf, Walter Dr, 67157 Wachenheim (DE); Peters, Jarren Dr, 68163 Mannheim (DE); Morsbach, Bernd, 67069 Ludwigshafen (DE); Kummer, Matthias Dr, 67273 Weisenheim (DE); Rühl, Thomas. Dr, 67227 Frankenthal (DE)

(56) Entgegenhaltungen:
- WO-A-01/14308
- US-A- 3 699 008
- US-A- 4 008 255
- US-A- 4 547 578

## Beschreibung

Die vorliegende Erfindung betrifft ein Verfahren zur Herstellung von spezifikationsgerechtem Phthalsäureanhydrid durch destillative Reinigung von rohem Phthalsäureanhydrid, wobei man rohes Phthalsäureanhydrid einer Destillationskolonne, die bei vermindertem Druck betrieben wird, zuführt, die Leichtsieder am Kopf oder in der Nähe des Kopfes der Destillationskolonne und das spezifikationsgerechte Phthalsäureanhydrid über einen Seitenabzug aus der Kolonne entnimmt.

Phthalsäureanhydrid ist eine bedeutsame Grundchemikalie der chemischen Industrie. Es dient zu einem beträchtlichen Teil als Ausgangsstoff für Dialkylphthalate, die in großen Mengen als Weichmacher für Kunststoffe wie PVC zum Einsatz kommen. Rohes Phthalsäureanhydrid wird in der Technik aus Naphthalin und/oder o-Xylol durch katalytische Oxidation in der Gasphase hergestellt. Vorzugsweise setzt man für die oben genannten Zwecke ein Phthalsäureanhydrid ein, das aus o-Xylol hergestellt wurde. Die Austräge der genannten gebräuchlichen Herstellverfahren weisen, bezogen auf ihr Gesamtgewicht, üblicherweise mehr als 99,5 Gew.-% Phthalsäureanhydrid auf. Das Phthalsäureanhydrid wird meist in flüssiger Form oder als Feststoff anhand von Abscheidern isoliert.

In Abhängigkeit vom gewählten Herstellverfahren und dabei besonders von den Ausgangsstoffen und den Katalysatoren enthält das Produkt ein jeweils spezifisches Spektrum von Verunreinigungen und Nebenprodukten (vgl. z.B.: H. Suter: "Wissenschaftliche Forschungsberichte: II. Anwendungstechnik und angewandte Wissenschaft", Dr. Dietrich Steinkopff Verlag, Darmstadt, 1972, Seite 39 ff.; im Folgenden kurz "Suter" genannt).

Am Markt wird eine Phthalsäureanhydrid-Qualität mit folgenden Spezifikationsgrenzen erwartet:

| | |
|---|---|
| Erstarrungspunkt (°C) | min. 130,8 |
| Massenanteile (Gew.-%): | |
| - PSA | min. 99,8 |
| - MSA | max. 0,05 |
| - Benzoesäure | max. 0,10 bzw. |
| | max. 0,002 bei Riechstoffqualität |
| - Phthalsäure | max. 0,1 |
| Schmelzfarbzahl (Hazen) | max. 20 |
| Hitzefarbzahl (Hazen) | max. 40 |

In der Technik hat sich über die lange Zeit, in der Phthalsäureanhydrid bereits im industriellen Maßstab hergestellt wird, die Abtrennung dieser Nebenprodukte mittels Destillation durchgesetzt (vgl. z.B.: "Ullmann's Encyclopedia of Industrial Chemistry", 5. Edition, Vol. A20, VCH Verlagsgesellschaft mbH, Weinheim, 1992, Seiten 181 bis 189; im Folgenden kurz "Ullmann" genannt; Kirk-Othmer "Encyclopedia of Chemical Technology", 4. Edition, Vol. 18, John Wiley & Sons, New York, 1996, Seiten 997 bis 1006, im Folgenden kurz "Kirk-Othmer" genannt). Niedrig siedende und/oder azeotrop destillierende Verunreinigungen, teilweise mit intensiver Eigenfarbe, bereiten dem Fachmann trotz vergleichsweise geringer Anteile dabei jedoch große Mühe.

Die Destillation - vor allem ihre unter wirtschaftlichen Aspekten häufig besonders interessante kontinuierliche Durchführung - erfolgt üblicherweise mittels zweier Kolonnen, um ein hinreichend reines Phthalsäureanhydrid zu erhalten. Im ersten Schritt werden dabei in der Regel die Leichtsieder (beispielsweise die Hauptmengen an Benzoesäure, Maleinsäureanhydrid, Citraconsäureanhydrid), also Stoffe mit einem Siedepunkt unterhalb des Siedepunktes des Phthalsäureanhydrids abgetrennt; in einem zweiten Schritt destilliert man dann Phthalsäureanhydrid von den Schwersiedern (beispielsweise Phthalsäure, bestimmte farbgebende Komponenten, Kondensationsprodukte aus Inhaltsstoffen des rohen Phthalsäureanhydrids), also Stoffen mit höheren Siedepunkten als dem von Phthalsäureanhydrid bzw. von undestillierbaren Bestandteilen, ab.

In "Suter" (dort auf Seite 45) wird bereits auf eine einstufige kontinuierliche Destillation von Phthalsäureanhydrid verwiesen (Ruhröl, Europa-Chemie 21, 7 (1965)), wobei keine weiteren Einzelheiten genannt werden.

Die US-A 4,547,578 (D2) offenbart ein Verfahren zur Entfernung von Naphthochinon aus rohem Phthalsäureanhydrid, welches durch Oxidation von Naphthalin hergestellt wurde. Dabei wird das rohe Phthalsäureanhydrid bei erhöhter Temperatur mit einem teilungesättigten aliphatischen Öl behandelt. Es schließt sich eine Destillation im Vakuum zur Entfernung des Öls und von Verun-reinigungen an. Das Rücklaufverhältnis in der Destillationskolonne liegt dabei insbe-sondere bei 2 : 1.

Aus der US-A 4,008,255 (D3) ist ein Verfahren zur Isolierung von Phthalsäurean-hydrid aus dem Reaktionsgas der katalytischen Oxidation von o-Xylol bekannt. Aus den zur Isolierung verwendeten Paraffinen wird das Phthalsäureanhydrid mecha-nisch als Maische abgetrennt. Die Maische wird danach destillativ in Phthalsäureanhydrid und Paraffine aufge-trennt, wobei das Phthalsäureanhydrid über einen Seitenabzug der Destillationsko-lonne abgezogen wird. Für die Herstellung reinerer Ware wird eine weitere übliche Destillation benötigt (Spalte 4, Zeilen 12-14).

Besonders höhe Anforderungen werden an solche aus Phthalsäureanhydrid synthetisierte Ester der Phthalsäure gestellt, welche als Lösungs- oder Streckmittel in Parfümen oder Kosmetika verwendet werden sollen. Die Anwesenheit von geringen Mengen Maleinsäure, Citraconsäure sowie deren Anhydriden und vor allem von Benzoesäure im Phthalsäureanhydrid führt jedoch zu Veresterungsprodukten dieser Stoffe mit intensiven charakteristischen Geruchsnoten, etwa einer diffus-fruchtigen Note im Falle von Benzoesäureethylester. Derartiger Verunreinigungen sind üblicherweise im Anschluss an die Ester-Synthese mittels eines kombinierten Wasch- und Extraktionsschrittes zu entfernen. Dieses Verfahren ist jedoch sehr aufwendig, macht aber andererseits normalerweise die vorangehende übliche Destillation des rohes Phthalsäureanhydrids nicht entbehrlich.

Die Aufgabe, die sich aus den Verunreinigungen des Phthalsäureanhydrids ergibt, lässt sich andererseits auch nicht befriedigend mit dem destillativen Verfahren lösen, welches aus der Anmeldung Wo-A 01/14308 bekannt ist. Dort wird spezifikationsgerechtes Phthalsäureanhydrid durch destillative Reinigung von rohem Phthalsäureanhydrid gewonnen, indem man rohes Phthalsäureanhydrid einer Destillationskolonne, die bei vermindertem Druck betrieben wird, zuführt, die Leichtsieder am Kopf oder in der Nähe des Kopfes der Destillationskolonne und das spezifikationsgerechte Phthalsäureanhydrid über einen Seitenabzug aus der Kolonne entnimmt.

In der Regel ist zur Abreicherung der genannten störenden Begleitstoffe vielmehr bisher ein kombinierter Wasch- und Extraktionsschritt erforderlich.

Der vorliegenden Erfindung lag daher die Aufgabe zugrunde, ein technisch einfaches und damit wirtschaftliches Verfahren zu finden, mit dem rohes Phthalsäureanhydrid so gereinigt werden kann, dass die am Markt verlangten niedrigen Gehalte an solchen Nebenkomponenten, insbesondere Benzoesäure, erreicht werden, welche bei der weiteren Umsetzung von Phthalsäureanhydrid zu Estern der Phthalsäure geruchsintensive Produkte bilden.

Demgemäß wurde ein Verfahren zur Herstellung von spezifikationsgerechtem Phthalsäureanhydrid durch destillative Reinigung von rohem Phthalsäureanhydrid gefunden, bei dem man rohes Phthalsäureanhydrid einer Destillationskolonne, die bei vermindertem Druck betrieben wird, zuführt, die Leichtsieder am Kopf oder in der Nähe des Kopfes der Destillationskolonne und das spezifikationsgerechte Phthalsäureanhydrid über einen Seitenabzug aus der Kolonne entnimmt, welches dadurch gekennzeichnet ist, dass man die Kolonne bei einem Rücklaufverhältnis x mit 1 : 1,8 < x < 1 : 2 betreibt.

Mit dem erfindungsgemäßen Verfahren ist es möglich, ein Phthalsäureanhydrid mit einem Gehalt an Benzoesäure von weniger als 20 ppm, vorzugsweise zwischen 5 und weniger als 20 ppm, und daneben einer Schmelzfarbzahl von weniger als 10 APHA und einer Hitzefarbzahl von weniger als 20 APHA herzustellen.

Geeignete Destillationskolonnen (im Folgenden auch kurz "Kolonnen" genannt) im Sinne der vorliegenden Erfindung sind Bodenkolonnen, Füllkörperkolonnen und Packungskolonnen sowie Kolonnen, in denen die technischen Merkmale dieser Kolonnentypen kombiniert wurden. Bevorzugt verwendet man Bodenkolonnen. Es sind bei den genannten Kolonnentypen übliche Einbauten wie handelsübliche Böden, Füllkörper oder Packungen, beispielsweise Glockenböden, Tunnelböden, Ventilböden, Siebböden, Dualflowböden und Gitterböden, Pall-Ringe®, Berl®-Sattelkörper, Netzdrahtringe, Raschig-Ringe®, Intalox®-Sättel, Interpak®-Füllkörper und Intos® aber auch geordnete Packungen, wie beispielsweise Sulzer-Mellapak®, Sulzer-Optiflow®, Kühni-Rombopak® und Montz-Pak® sowie Gewebepackungen verwendbar. Im Bereich unterhalb des Zulaufes der Kolonne werden vorzugsweise Einbauten gewählt, die auch feststofftauglich sind, insbesondere Dual-Flow-Böden. Geeignet sind hierfür in der Regel Böden und Füllkörper der oben genannten Bauarten.

Die Kolonne ist in der Regel mit einem Sumpfverdampfer sowie mit einem Kondensator am Kolonnenkopf ausgestattet.

Der Durchmesser der Kolonne richtet sich nach den jeweils angestrebten Durchsätzen und kann nach den gängigen Regeln der Technik vom Fachmann problemlos ermittelt werden.

Die Höhe der Kolonne sowie die Lagen von Zulauf und Seitenabzug können mit dem Konzept der theoretischen Trennstufen in Verbindung mit den gewählten Einbauten ermittelt werden.

Als eine theoretische Trennstufe wird diejenige Kolonneneinheit definiert, die ein Anreichern der leichtflüchtigen Komponente entsprechend dem thermodynamischen Gleichgewicht bewirkt, vorausgesetzt, dass ideale Durchmischung vorliegt, beide Phasen im Gleichgewicht stehen und kein Mitreißen von Flüssigkeitstropfen erfolgt (vgl. Vauck, Müller: Grundoperationen chemischer Verfahrenstechnik, VCH Verlagsgesellschaft mbH, Weinheim, 1988).

Im Allgemeinen ist die erfindungsgemäße Kolonne in drei Abschnitte unterteilt, die durch die Lagen von Zulauf, Seitenabzug, Kopf und Sumpf festgelegt werden. Die Zahl der theoretischen Trennstufen für die beiden oberen Abschnitte wird nach üblichen verfahrenstechnischen Überlegungen in Abhängigkeit vom Leichtsiederanteil im rohen Phthalsäureanhydrid und dem gewünschten Restgehalt an Leichtsiedern im gereinigten Phthalsäureanhydrid festgelegt. Die Zahl der theoretischen Trennstufen für den unteren Abschnitt der erfindungsgemäßen Kolonne beträgt im Allgemeinen 1 bis 10, vorzugsweise 2 bis 8 und vor allem 3 bis 7.

Besonders eignet sich das erfindungsgemäße Verfahren für rohes Phthalsäureanhydrid, wie es durch katalytische Gasphasenoxidation von o-Xylol erhalten wird und welches vorzugsweise mehr als 95 und insbesondere mehr als 98 Gew.-% Phthalsäureanhydrid aufweist.

Die Kolonne wird vorzugweise bei einem absoluten Druck am Kolonnenkopf von 0,05 bis 0,5, bevorzugt 0,1 bis 0,3, besonders bevorzugt bei 0,15 bis 0,25 bar betrieben. Die Temperaturen in der Kolonne liegen am Kolonnenkopf im Allgemeinen bei 190 bis 220, vorzugsweise bei 196 bis 202 und vor allem bei 198 bis 200°C und am Kolonnensumpf bei 220 bis 260, vorzugsweise bei 230 bis 250 und vor allem bei 235 bis 245°C. Die Temperatur am Seitenabzug liegt im Allgemeinen bei 190 bis 250 und vorzugsweise bei 220 bis 240°C.

Die Destillation kann diskontinuierlich und, vorzugsweise, kontinuierlich durchgeführt werden. Das rohe Phthalsäureanhydrid wird der Kolonne vorzugsweise gasförmig und besonders bevorzugt flüssig zugeführt. Die Entnahme in flüssiger Form erfolgt normalerweise oberhalb des Zulaufs der Kolonne, die Entnahme im gasförmigen Zustand, die bevorzugt ist, erfolgt normalerweise unterhalb des Zulaufs.

In einer bevorzugten Ausführungsform des erfindungsgemäßen Verfahrens bei Verwendung von Bodenkolonnen können technische Einrichtungen wie Tropfenabscheider an dem Seitenabzug innerhalb oder außerhalb der Kolonne angebracht werden.

Der Sumpfverdampfer der Kolonne wird vorzugsweise als Fallfilmverdampfer ausgestaltet. Fallfilmverdampfer sind in der Verfahrenstechnik allgemein bekannt. Sie haben die Vorteile einer geringeren mittleren Verweilzeit der Flüssigkeit im Verdampfungsbereich mit der Folge einer schonenderen Verdampfung gegenüber Zwangsumlaufentspannungsverdampfern. Durch die schonendere Behandlung kann die Neigung zur Feststoffbildung vermindert werden, die geringen Verweilzeiten führen zu einer Reduzierung von unerwünschten Nebenreaktionen und somit zu einer verbesserten Ausbeute im Sumpf, und die Betriebskosten können in aller Regel dadurch gesenkt werden.

Das spezifikationsgerechte Phthalsäureanhydrid wird üblicherweise unmittelbar nach der Entnahme aus der Kolonne gekühlt und als Flüssigkeit oder, nach dem Erstarren, als Festkörper erhalten. Ein noch höherer Reinheitsgrad kann gewünschtenfalls erreicht werden, indem man das Phthalsäureanhydrid beispielsweise über eine Seitenkolonne feindestilliert oder ein Trennblech achsial über einen bestimmten Bereich in der Kolonne (sog. Petlyuk-Verschaltung) anbringt. Auch ein Umkristallieren kommt hier in Betracht.

Über den Seitenabzug wird das spezifikationsgerechte Phthalsäureanhydrid normalerweise in einer Menge von mindestens 97 Gew.-% bezogen auf das Gewicht des Zulaufs abgenommen. Bei kontinuierlichem Betrieb der Kolonne liegt die entnommene Menge vorzugsweise bei mindestens 90, besonders bevorzugt bei mindestens 95 Gew.-%, bezogen auf das Gewicht des Zulaufs.

Die Wiedergewinnungsrate an Phthalsäureanhydrid bezogen auf den Gehalt im Zustrom zur Kolonne liegt in der Regel bei 98 % und höher.

Die bei der Destillation anfallenden Leichtsieder sowie Hochsieder werden üblicherweise in die Verbrennung gegeben.

Es versteht sich, dass das erfindungsgemäße Verfahren für individuelle Trennprobleme noch optimal angepasst werden kann. Diese Anpassung kann der Fachmann routinemäßig vornehmen, wenn er der hierin offenbarten Lehre folgt.

Die Reinheit des so erhaltenen Phthalsäureanhydrids läßt sich anhand allgemein bekannter analytischer Methoden wie der Gaschromatographie, der UV-Spektroskopie und der Säure-Base-Titration ermitteln. Weil für die meisten Verwendungszwecke ein Phthalsäureanhydrid ohne verfärbende Verunreinigungen benötigt wird, kommt der Charakterisierung durch die sogenannten Farbzahlen - vorrangig der Schmelzfarbzahl und der Hitzefarbzahl - besondere Bedeutung zu. Farbliche Veränderungen des Phthalsäureanhydrids unter thermischer Belastung sind deswegen von praktischer Bedeutung, weil Phthalsäureanhydrid normalerweise im geschmolzenen Zustand - etwa bei 160°C - gelagert und transportiert wird. Insbesondere die Schmelzfarbzahl (APHA/Hazen-Farbskala, vgl. W. Liekmeier, D. Thybusch: Charakterisierung der Farbe von klaren Flüssigkeiten, Editor: Bodenseewerk Perkin-Elmer GmbH, Überlingen, 1991) wird im Allgemeinen so ermittelt, dass man die Farbzahl von Phthalsäureanhydrid unmittelbar nach der Probenahme bei einer Temperatur von 160°C bestimmt. Weiterhin wird die Hitzefarbzahl im Allgemeinen so ermittelt, dass man das Phthalsäureanhydrid 90 Minuten lang bei 250°C hält und dann die Farbzahl misst.

Die Bestimmung des Gehaltes an Benzoesäure in Phthalsäureanhydrid ist mittels herkömmlicher quantitativer Gaschromatographie möglich.

Mit dem erfindungsgemäßen Verfahren kann der Gehalt des Phthalsäureanhydrids an den oben genannten unerwünschten leicht-flüchtigen Begleitstoffen, vor allem Benzoesäure, auf Werte von weniger als 20 Gewichts-ppm abgesenkt werden, dass das erhaltene Phthalsäureanhydrid nunmehr auch höchsten Anforderungen der Riechstoff- und Kosmetikindustrie genügt.

### Beispiel

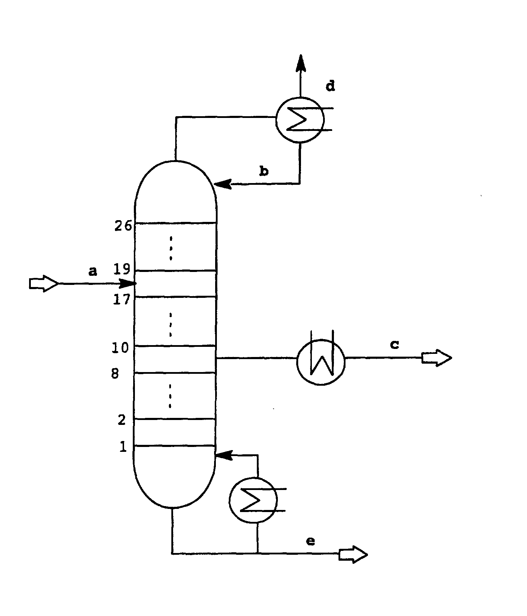

Es kam eine Bodenkolonne gemäß der schematischen Abbildung 1 zum Einsatz. Die Kolonne wies 26 Ventilböden (überschlagsmäßig 16 theoretische Trennstufen) auf und hatte einen Durchmesser von 50 mm. Der Seitenabzug befand sich zwischen dem 8. und dem 10. Boden über dem Sumpf (überschlagsmäßig im Bereich zwischen der fünften und der sechsten theoretischen Trennstufe), der Zulauf befand sich zwischen dem 17. und dem 19. Boden über dem Sumpf (überschlagsmäßig im Bereich der elften theoretischen Trennstufe). In Abbildung 1 sind weiterhin noch der 1. und der 2. Boden eingezeichnet, die übrigen Böden sind durch senkrechte unterbrochene Linien angedeutet.

Es kam ein rohes Phthalsäureanhydrid zur Destillation, das durch Gasphasenoxidation von o-Xylol an einem Festbett in Gegenwart eines Katalysators, bestehend aus einem Trägerkern und den darauf schalenförmig aufgebrachten katalytisch wirksamen Metalloxiden Cäsiumoxid (berechnet als 0,4 Gew.-% Cäsium), Vanadiumoxid (4 Gew.-%) und Titandioxid (95,6 Gew.-%), hergestellt worden war (vgl. die ältere deutsche Patentanmeldung mit dem Aktenzeichen 198 24 532). Die Beladung im Reaktor betrug 86 g o-Xylol pro Nm³ Luft. Die Reaktortemperatur lag zwischen 350 und 450°C.

Das so erhaltene rohe Phthalsäureanhydrid hatte folgende gewichtsbezogene Zusammensetzung:

| | |
|---|---|
| 99,24 Gew.-% | Phthalsäureanhydrid |
| 0,2 Gew.-% | Benzoesäure |
| 200 ppm | Maleinsäureanhydrid |
| 20 ppm | Citraconsäureanhydrid |
| 0,5 Gew.-% | Phthalsäure |

und der Rest zu 100 Gew.-% sonstige Stoffe.

1000 g dieses rohen Phthalsäureanhydrids wurden der Kolonne stündlich kontinuierlich zugeführt (a). Bei einem Rücklauf von 530 g (b) (Rücklaufverhältnis 1 : 1,88), einem absoluten Druck von 0,17 bar am Kolonnenkopf, einer Temperatur von 198°C am Kolonnenkopf und 238°C am Kolonnensumpf wurden in der gleichen Zeit 970 g gereinigtes Phthalsäureanhydrid über den Seitenabzug bei 221°C entnommen, kondensiert und isoliert (c). Der Kopfabzug über (d) wurde dabei in eine Kühlfalle kondensiert und betrug ca. 7 g; der Sumpfabzug über (e) betrug ca. 15 g und enthielt die Schwersieder und nichtdestillierbare Anteile. Die Analyse des über den Seitenabzug bei (c) isolierten Phthalsäureanhydrids ergab folgende gewichtsbezogene Zusammensetzung:

| | |
|---|---|
| 99,97 Gew.-% | Phthalsäureanhydrid |
| 15 ppm | Benzoesäure |
| < 10 ppm | Maleinsäureanhydrid |
| < 10 ppm | Citraconsäureanhydrid |
| 0,02 Gew.-% | Phthalsäure |

und der Rest zu 100 Gew.-% sonstige Stoffe.

Die Schmelzfarbzahl wurde unmittelbar nach der Entnahme ermittelt und betrug 5-10 APHA. Die Hitzefarbzahl wurde wie folgt ermittelt: Eine Probe Phthalsäureanhydrid wurde in einem Wärmeschrank 1,5 Stunden bei einer Temperatur von 250°C getempert. Anschließend wurde die Farbzahl zu 10-20 APHA gemessen.

## Patentansprüche

1. Verfahren zur Herstellung von spezifikationsgerechtem Phthalsäureanhydrid durch destillative Reinigung von rohem Phthalsäureanhydrid, wobei man rohes Phthalsäureanhydrid einer Destillationskolonne, die bei vermindertem Druck betrieben wird, zuführt, die Leichtsieder am Kopf oder in der Nähe des Kopfes der Destillationskolonne und das spezifikationsgerechte Phthalsäureanhydrid über einen Seitenabzug aus der Kolonne entnimmt, **dadurch gekennzeichnet, dass** man die Kolonne bei einem Rücklaufverhältnis x mit 1 : 1,8 < x < 1 : 2 betreibt.

2. Verfahren nach Anspruch 1, **dadurch gekennzeichnet, dass** man die Destillation bei einem Druck am Kolonnenkopf von 0,05 bis 0,5 bar durchführt.

3. Verfahren nach Anspruch 1 oder 2, **dadurch gekennzeichnet, dass** sich der Seitenabzug näher am oberen der beiden Böden befindet, zwischen denen er angeordnet ist.

4. Verfahren nach den Ansprüchen 1 bis 3, **dadurch gekennzeichnet, dass** man der Kolonne das spezifikationsgerechte Phthalsäureanhydrid über den Seitenabzug gasförmig entnimmt.

## Claims

1. A process for the preparation of on-spec phthalic anhydride by distillative purification of crude phthalic anhydride, where crude phthalic anhydride is passed to a distillation column which is operated at reduced pressure, the low-boiling components are removed at the top or in the vicinity of the top of the distillation column and the on-spec phthalic anhydride is removed from the column via a side take-off, which comprises operating the column at a reflux ratio x where 1 : 1.8 < x < 1 : 2.

2. A process as claimed in claim 1, wherein the distillation is carried out at a pressure at the top of the column of from 0.05 to 0.5 bar.

3. A process as claimed in claim 1 or 2, wherein the side take-off is located closer to the upper of the two trays between which it is arranged.

4. A process as claimed in any of claims 1 to 3, wherein the on-spec phthalic anhydride is removed from the column in gaseous form via the side take-off.

## Revendications

1. Procédé pour la préparation de l'anhydride phtalique selon la description, au moyen d'une rectification de l'anhydride phtalique brut, dans lequel on amène de l'anhydride phtalique dans une colonne de distillation fonctionnant sous pression réduite, et dans lequel on soutire, au niveau de la tête ou près de la tête de la colonne de distillation, les produits à faible point d'ébullition, et, par une prise latérale de la colonne, l'anhydride phtalique selon la description, **caractérisé en ce que** l'on fait fonctionner la colonne à un taux de reflux x satisfaisant la relation 1:1,8 < x < 1:2.

2. Procédé selon la revendication 1, **caractérisé en ce que** l'on réalise la distillation sous une pression, au niveau de la tête de la colonne, allant de 0,05 bar à 0,5 bar.

3. Procédé selon la revendication 1 ou 2, **caractérisé en ce que** la prise latérale, située entre deux plateaux, est disposée plus près du plateau supérieur.

4. Procédé selon l'une quelconque des revendications 1 à 3, **caractérisé en ce que** l'on soutire, par la prise latérale de la colonne, l'anhydride phtalique selon la description sous forme gazeuse.
